(19) Europäisches Patentamt

European Patent Office

Office européen des brevets

(11) **EP 1 683 861 A1**

(12) **DEMANDE DE BREVET EUROPEEN**

(43) Date de publication:
**26.07.2006 Bulletin 2006/30**

(21) Numéro de dépôt: **05291780.4**

(22) Date de dépôt: **11.08.1999**

(51) Int Cl.:
*C12N 15/10* (2006.01)   *C12N 15/31* (2006.01)
*C12N 1/21* (2006.01)   *C07K 14/00* (2006.01)

(84) Etats contractants désignés:
**AT BE CH CY DE DK ES FI FR GB GR IE IT LI LU MC NL PT SE**

(30) Priorité: **12.08.1998 FR 9810338**

(62) Numéro(s) de document de la (des) demande(s) initiale(s) en application de l'article 76 CBE:
**99936725.3 / 1 104 457**

(71) Demandeur: **Proteus**
**30000 Nîmes (FR)**

(72) Inventeurs:
• **Dupret, Daniel**
**30420 Calvisson (FR)**
• **Masson, Jean-Michel**
**31400 Toulouse (FR)**

• **Lefevre, Fabrice**
**32120 Bajonnette (FR)**

(74) Mandataire: **Breese Derambure Majerowicz**
**38, avenue de l'Opéra**
**75002 Paris (FR)**

Remarques:
•Cette demande a été déposée le 25 - 08 - 2005 comme demande divisionnaire de la demande mentionnée sous le code INID 62.
•Le listage des séquences, qui est publié en annexe aux documents de la demande, a été produit après la date de dépôt. Le demandeur a déclaré qu'il ne contient pas d'élément s'étendant au-delà du contenu de la demande telle qu'elle a été déposée.

(54) **Procédé d'obtention in vitro de séquences polynucléotidiques recombinées, banques de séquences et séquences ainsi obtenues**

(57) La présente invention a pour objet un procédé de production *in vitro* de séquences polynucléotidiques recombinées à partir d'une banque de séquences polynucléotidiques, caractérisé en ce qu'il comprend les étapes suivantes : (a) la fragmentation d'une banque initiale de séquences polynucléotidiques double-brins, (b) la dénaturation des fragments issus de l'étape (a) éventuellement en présence d'une ou plusieurs matrice(s) d'assemblage, (c) l'hybridation desdits fragments avec une ou plusieurs matrice(s) d'assemblage si celle(s)-ci n'est (ne sont) pas présente(s) à l'étape (b), (d) la ligation desdits fragments, (e) éventuellement le clonage des séquences polynucléotidiques recombinées, et la sélection des séquences polynucléotidiques présentant des propriétés avantageuses par rapport à une ou plusieurs séquences de référence.

**EP 1 683 861 A1**

**EP 1 683 861 A1**

SEQUENCE LISTING

<110> PROTEUS SA
<120> Procédé d'obtention in vitro de séquences polynucléotidiques recombinées, banques de séquences et séquences ainsi obtenues
<130> 1246/EP

<140> EP05291780.4
<141> 2005-08-25

<150> EP99936725.3
<151> 1999-08-11

<150> PCT/FR99/01973
<151> 1999-08-11

<150> FR 98/10338
<151> 1998-08-12

<160> 6

<170> PatentIn version 3.1

<210> 1
<211> 33
<212> DNA
<213> artificial sequence

<220>
<223> Oligo N

<400> 1

actgactacc atggccggga atgaccgcga gcc                33


<210> 2
<211> 39
<212> DNA
<213> artificial sequence

<220>
<223> Oligo E

<400> 2

ccgcggtgga gcgaattcta attactacca aacatatcc          39


<210> 3
<211> 26

<212> DNA
<213> artificial sequence

<220>
<223> Oligo M1

<400> 3

gcgcctgaat attgcggaga aaaagc                    26


<210> 4
<211> 30
<212> DNA
<213> artificial sequence

<220>
<223> Oligo M2

<400> 4

acaaccagat gaaaagaaag ggttaatatc                30


<210> 5
<211> 16
<212> DNA
<213> artificial sequence

<220>
<223> Oligo A1

<400> 5

actgactacc atggcc                               16


<210> 6
<211> 18
<212> DNA
<213> artificial sequence

<220>
<223> Oligo A2

<400> 6

ccgcggtgga gcgaattc                             18

SEQUENCE LISTING

<110> PROTEUS SA
<120> Procédé d'obtention in vitro de séquences
polynucléotidiques recombinées, banques de séquences et
séquences ainsi obtenues
<130> 1246/EP

<140> EP05291780.4
<141> 2005-08-25

<150> EP99936725.3
<151> 1999-08-11

<150> PCT/FR99/01973
<151> 1999-08-11

<150> FR 98/10338
<151> 1998-08-12

<160> 6

<170> PatentIn version 3.1

<210> 1
<211> 33
<212> DNA
<213> unidentified

<220>
<221> oligonucléotide
<222> (1)..(33)
<223> Oligo N

<400> 1

actgactacc atggccggga atgaccgcga gcc                33

<210> 2
<211> 39
<212> DNA
<213> unidentified

<220>
<221> oligonucléotide
<222> (1)..(39)
<223> Oligo E

<400> 2

ccgcggtgga gcgaattcta attactacca aacatatcc          39

```
<210>  3
<211>  26
<212>  DNA
<213>  unidentified

<220>
<221>  oligonucléotide
<222>  (1)..(26)
<223>  Oligo M1

<400>  3

gcgcctgaat attgcggaga aaaagc                    26


<210>  4
<211>  30
<212>  DNA
<213>  unidentified

<220>
<221>  oligonucléotide
<222>  (1)..(30)
<223>  Oligo M2

<400>  4

acaaccagat gaaaagaaag ggttaatatc                30


<210>  5
<211>  16
<212>  DNA
<213>  unidentified

<220>
<221>  oligonucléotide
<222>  (1)..(16)
<223>  Oligo A1

<400>  5

actgactacc atggcc                               16


<210>  6
<211>  18
<212>  DNA
<213>  unidentified



<220>
<221>  oligonucléotide
<222>  (1)..(18)
<223>  Oligo A2

<400>  6

ccgcggtgga gcgaattc                             18
```

**Description**

La présente invention se rapporte à une méthode d'obtention *in vitro* de séquences polynucléotidiques recombinées. L'invention vise tout particulièrement à générer puis sélectionner des séquences polynucléotidiques susceptibles de présenter une ou plusieurs propriétés avantageuses par rapport aux propriétés correspondantes de séquences de référence et donc capables de conférer un phénotype amélioré et/ou de produire des protéines améliorées.

On entend par séquence de référence une séquence ayant des propriétés proches de celles recherchées.

Différentes techniques ont été développées pour favoriser la recombinaison *in vitro* entre différentes séquences polynucléotidiques, parmi celles-ci on peut citer plus particulièrement le DNA-Shuffling (12) et le StEP (14) toutes deux fondées sur l'utilisation de la PCR.

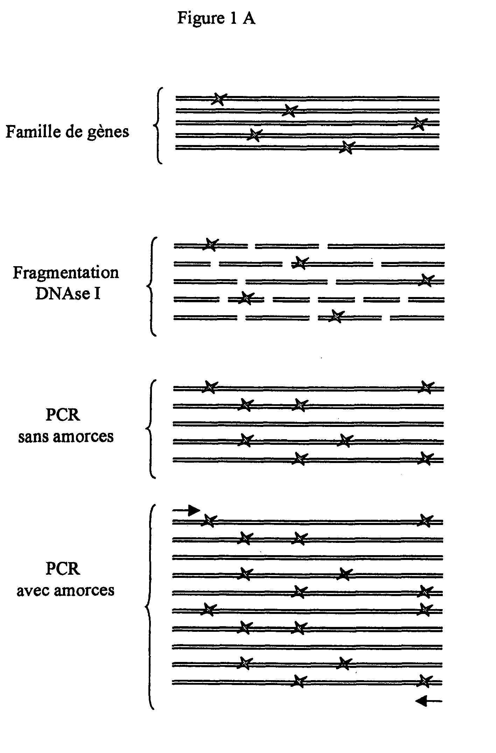

Le DNA-Shuffling comporte deux étapes, la fragmentation aléatoire par la DNAse I de séquences polynucléotidiques, et une amplification par PCR dans laquelle les fragments précédemment générés servent d'amorces. A chaque étape d'hybridation, le changement de matrice provoque des recombinaisons au niveau des régions ayant des séquences homologues. Une représentation schématique de cette méthode est donnée à la figure 1A en annexe.

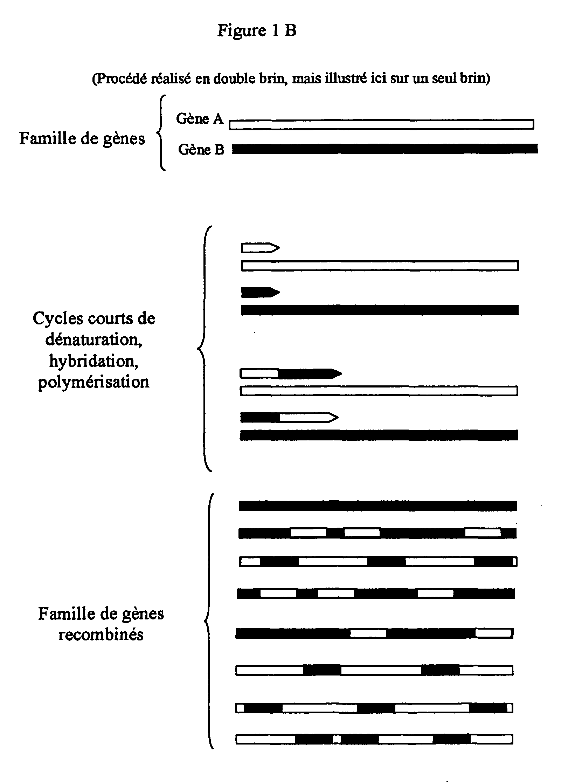

Le StEP consiste à mélanger différentes séquences polynucléotidiques contenant diverses mutations en présence d'une paire d'amorces. Ce mélange est soumis à une réaction de type PCR dans laquelle les étapes d'hybridation et de polymérisation sont regroupées en une seule étape de très courte durée. Ces conditions permettent l'hybridation des amorces mais diminuent la vitesse de polymérisation, de façon à ce que les fragments qui sont partiellement synthétisés s'hybrident aléatoirement sur les séquences polynucléotidiques portant les différentes mutations, permettant ainsi la recombinaison. Une représentation schématique de cette méthode est donnée à la figure 1B en annexe.

Dans chacune de ces deux méthodes, l'étape de polymérisation est indispensable au processus de recombinaison. Ainsi, en fonction des polymérases choisies, cette étape de polymérisation peut être génératrice de mutations supplémentaires non souhaitées. En outre, à partir d'un certain nombre de cycles, le DNA-Shuffling et le StEP reposent sur le principe de l'hybridation d'une "méga-amorce"(6) sur une matrice, ce qui entraîne probablement des difficultés de mise en oeuvre pour des séquences polynucléotidiques dont la taille est supérieure à 1,5 Kpb (11). Enfin, ces deux techniques ne permettent pas de contrôler le taux de recombinaisons, puisque celles-ci se font aléatoirement au cours des étapes successives de polymérisation.

La présente invention vise précisément à palier les inconvénients ci-dessus en offrant une méthode simple de préparation de séquences polynucléotidiques recombinées, permettant de générer des séquences polynucléotidiques susceptibles de présenter des propriétés avantageuses par rapport aux propriétés correspondantes de séquences de référence et donc capables de conférer un phénotype amélioré et/ou de produire des protéines améliorées.

Ce but est atteint grâce à un procédé d'obtention *in vitro* de séquences polynucléotidiques recombinées à partir d'une banque de séquences polynucléotidiques, aussi désignée ci-après banque initiale, caractérisé en ce qu'il comprend les étapes suivantes :

a) la fragmentation d'une banque de séquences polynucléotidiques double-brins,
b) la dénaturation des fragments éventuellement en présence d'une ou plusieurs matrice(s) d'assemblage,
c) l'hybridation desdits fragments avec une ou plusieurs matrice(s) d'assemblage si celle(s)-ci n'est (ne sont) pas présente(s) à l'étape (b),
d) la ligation desdits fragments pour obtenir des séquences polynucléotidiques recombinées,
e) la sélection des séquences polynucléotidiques recombinées présentant des propriétés avantageuses par rapport aux propriétés correspondant à une ou plusieurs séquences de référence.

Le procédé de l'invention peut comprendre à l'issue de l'étape (d) et avant l'étape (e), la répétition des étapes (b), (c) et (d) non plus avec les fragments de l'étape (a) mais avec les produits de l'étape (d).

Ce mode de réalisation est particulièrement utile dans le cas où à l'issue de l'étape (d) tous les fragments ne sont pas ligués. Dans ce cas, le procédé de l'invention comprend en outre, à la fin de l'étape (d) et avant l'étape (e), un ou plusieurs cycles des réactions suivantes:

- dénaturation des fragments ligués et non ligués issus de l'étape (d), éventuellement en présence d'une ou plusieurs matrice(s) d'assemblage,
- hybridation desdits fragments avec une ou plusieurs matrice(s) d'assemblage si celle(s)-ci n'est (ne sont) pas présente(s) lors de la dénaturation,
- ligation desdits fragments.

Ces réactions de dénaturation, hybridation et ligation, sont équivalentes aux étapes (b), (c) et (d), mais réalisées non pas avec les fragments de l'étape (a) mais avec les fragments ligués et non ligués issus de l'étape (d).

Le procédé de l'invention peut comprendre en outre, une ou plusieurs des étapes suivantes :

2

- la séparation des séquences polynucléotidiques recombinées de la ou des matrice(s) d'assemblage avant l'étape (e),
- l'amplification des séquences polynucléotidiques recombinées double brin avant l'étape (e).
- le clonage de séquences polynucléotidiques recombinées éventuellement après séparation des brins recombinés de la ou des matrices et obtention du double brin correspondant avant l'étape (e).

Les extrémités des fragments générés à l'étape (a) sont telles qu'il puisse y avoir hybridation adjacente de ces extrémités sur la ou les matrice(s) d'assemblage à l'étape (c) et ligation de ces fragments les uns avec les autres à l'étape (d). Les séquences polynucléotidiques de la banque sur laquelle est effectué le procédé de l'invention doivent présenter des zones d'homologie soit entre elles, soit avec les matrices d'assemblage, permettant de générer des extrémités de fragments telles que décrites ci-dessus.

Une forme de mise en oeuvre avantageuse du procédé de l'invention consiste à réaliser simultanément les étapes (c) et (d) selon une réaction dite de RLR pour l'expression anglaise de "Recombining Ligation Reaction".

Outre les avantages indiqués précédemment, le procédé de l'invention est remarquable en ce qu'il favorise et accélère la recombinaison aléatoire *in vitro* de séquences polynucléotidiques, ces séquences polynucléotidiques pouvant être des gènes. On entend par gène un fragment ou une séquence d'ADN associée à une fonction biologique. Un gène peut être obtenu de différentes façons, dont la synthèse chimique, la synthèse par polymérisation ou par extraction dudit gène à partir d'une source d'acides nucléiques.

La recombinaison *in vitro* des séquences polynucléotidiques de la banque initiale par le procédé de l'invention permet donc d'obtenir une nouvelle banque contenant des séquences ayant acquis une ou plusieurs caractéristiques des séquences de la banque précédente. Le procédé de l'invention constitue donc une technique d'évolution *in vitro.*

Le procédé de l'invention constitue une alternative à la PCR recombinatoire telle que mise en oeuvre dans les techniques de DNA shuffling (12) ou de StEP (14), puisqu'il ne nécessite pas d'étape de polymérisation *in vitro* pour aboutir à la recombinaison. Au contraire, l'étape clef du procédé de l'invention est l'étape (d) de ligation sur une matrice d'assemblage, ce qui assure un très haut degré de fidélité au cours des événements de recombinaison.

Le procédé de l'invention est remarquable en ce qu'il permet d'augmenter considérablement l'efficacité du réassemblage des fragments à liguer. En effet, dans le cas d'une séquence découpée en n fragments, il existe $n^n$ possibilités de réassociation des fragments en utilisant un procédé de ligation classique (sans utilisation d'une matrice de réassemblage qui oriente la ligation), parmi lesquelles une seule forme est intéressante. Dans le cas du procédé de l'invention, la ligation est orientée par la matrice d'assemblage, ce qui permet d'obtenir directement la seule forme intéressante.

La fragmentation de ces séquences polynucléotidiques à l'étape (a) peut se faire soit de manière contrôlée, soit de manière aléatoire.

Dans le cas d'une fragmentation réalisée de manière contrôlée, la fragmentation permet de maîtriser avec précision le degré de recombinaison voulu et la position des points de recombinaison. Selon une forme de réalisation préférée du procédé de l'invention, l'étape (a) consiste à soumettre les séquences polynucléotidiques de la banque à une hydrolyse par l'action d'une ou plusieurs enzymes de restriction. Ainsi, dans une forme de mise en oeuvre particulière du procédé de l'invention, le degré de recombinaison et la position des points de recombinaison des séquences polynucléotidiques recombinées sont déterminés par la fragmentation de l'étape (a).

Ainsi, plus le nombre de fragments générés par séquence est grand, plus le nombre de fragments nécessaires pour recomposer une séquence est élevé, ce qui entraîne un taux de recombinaison élevé. En outre, la nature et la position des extrémités des fragments générés dans ce mode de réalisation du procédé de l'invention peuvent être connues et contrôlées, ce qui permet :

- de contrôler avec précision les zones où la recombinaison a lieu, ou
- d'induire la recombinaison entre des séquences polynucléotidiques, par exemple des gènes, si les extrémités des fragments sont créées dans des zones d'homologie entre ces séquences, ou des zones d'homologies entre ces séquences et la ou les matrices d'assemblage.

Dans le cas d'une fragmentation aléatoire, tout moyen enzymatique ou mécanique connu de l'homme de métier capable de couper aléatoirement l'ADN double brin pourra être utilisé, comme par exemple une digestion par la Dnase I ou une sonication.

Le procédé de l'invention permettant d'augmenter considérablement l'efficacité de réassemblage des fragments à liguer, il peut donc être appliqué à l'orientation de la ligation multi-moléculaire à bouts francs. Dans cette application, on utilise comme matrice d'assemblage aux étapes (b) ou (c) des oligonucléotides simple ou double brin juste complémentaires de l'extrémité 3' d'un fragment et 5' du fragment adjacent, ce qui permet l'hybridation adjacente de ces deux extrémités sur la même matrice après l'étape de dénaturation. Une fois hybridées, les extrémités des fragments peuvent être liguées entre-elles de façon à orienter le sens de ligation des fragments à bout francs. La même approche peut être envisagée pour l'orientation de la ligation de fragments à bouts cohésifs.

Une mise en oeuvre toute préférée du procédé de l'invention consiste à ajouter à l'étape (c) et/ou à l'étape (d) des enzymes capables de reconnaître et de couper de manière spécifique les extrémités non hybridées de fragments, lorsque celles-ci recouvrent d'autres fragments hybridés sur la même matrice. Un exemple préféré de ce type d'enzyme est l'enzyme Flap endonucléase (10).

Une mise en oeuvre particulière du procédé de l'invention consiste donc à utiliser des enzymes du type Flap endonucléases lorsque les fragments générés à l'étape (a) peuvent se recouvrir lors de l'hybridation sur la matrice d'assemblage à l'étape (c).

Ainsi, lors de l'hybridation de fragments d'ADN simple brin sur une matrice, ces enzymes ont pour propriété de reconnaître et de couper de manière spécifique les extrémités non hybridées de ces fragments, lorsque celles-ci recouvrent d'autres fragments hybridés sur la même matrice. Au cours de l'étape (c) d'hybridation, ces enzymes permettent donc d'augmenter le nombre d'extrémités adjacentes pouvant être liguées à l'étape (d), ce qui est particulièrement important dans le cas de fragments obtenus par coupure aléatoire, car ces fragments présentent des zones de recouvrement les uns avec les autres lorsqu'ils s'hybrident sur la matrice d'assemblage.

Dans une mise en oeuvre particulière du procédé de l'invention utilisant une ligase active à haute température et préférentiellement thermostable à l'étape (d), les endonucléases capables de reconnaître et de couper de manière spécifique les extrêmités non hybridées des fragments comme les Flaps, ajoutées à l'étape (c) et/ou à l'étape (d) auront les mêmes propriétés de thermorésistance et d'activité à haute température que ladite ligase.

La banque de séquences polynucléotidiques sur laquelle est effectuée le procédé de l'invention peut être générée par toute méthode connue de l'homme du métier, par exemple à partir d'un gène sauvage par étapes de mutagénèse dirigée successives, par PCR "error prone " (2), par mutagénèse aléatoire chimique, par mutagénèse aléatoire *in vivo,* ou en combinant des gènes de familles proches ou distinctes au sein de la même espèce ou d'espèces différentes de façon à disposer d'une variété de séquences polynucléotidiques dans ladite banque.

Parmi ces techniques, l'invention envisage plus particulièrement, un procédé dans lequel la banque de séquences polynucléotidiques double-brins est obtenue par une réaction de polymérisation en chaîne réalisée dans des conditions qui permettent de créer des mutations ponctuelles aléatoires.

La banque initiale de séquences polynucléotidiques double-brins peut être constituée de séquences synthétiques qui seront fragmentées à l'étape (a) ou qui peuvent constituer les fragments de l'étape (a)

Selon une forme de réalisation préférée du procédé de l'invention, l'étape (a) consiste à soumettre les séquences polynucléotidiques de la banque à une hydrolyse par l'action d'une ou plusieurs enzymes de restriction.

Pour accroître le degré de recombinaison généré par le procédé de l'invention, il suffit d'augmenter le nombre de fragments de restriction en utilisant des enzymes de restriction ayant un grand nombre de sites de coupure sur les séquences polynucléotidiques de la banque, ou en combinant plusieurs enzymes de restriction. Dans le cas de l'utilisation d'une ligase thermostable et thermoactive, la taille du plus petit fragment ainsi généré sera avantageusement supérieure ou égale à 40 pb, afin de conserver une température d'hybridation compatible avec celle de l'étape (d) de ligation qui est généralement de l'ordre de 65 °C.

L'étape (a) peut encore être réalisée en générant une banque de fragments par traitement aléatoire enzymatique ou mécanique. En particulier, l'étape (a) peut consister en un traitement aléatoire avec la DNAse I d'une banque de séquences polynucléotidiques double brins partiellement hétérologues. Dans le cas où l'on utilise à l'étape (a) une fragmentation enzymatique ou mécanique aléatoire, cette forme de mise en oeuvre du procédé de l'invention a la particularité de permettre l'utilisation des fragments générés par ce traitement comme matrices les uns pour les autres, pour l'hybridation au cours de l'étape (c) ou de la réaction de RLR des étapes (c) et (d) simultanées.

L'étape (b) peut être réalisée en combinant au moins deux banques de fragments distinctes générées séparément à l'étape (a) à partir de la même banque initiale par des traitements différents, comme par exemple avec des enzymes de restriction différentes. Dans le cas de la mise en oeuvre de telles banques, on utilise les fragments obtenus à l'étape (a) comme matrices les uns pour les autres, pour l'hybridation au cours de l'étape (c) ou de la réaction de RLR des étapes (c) et (d) simultanées.

Les fragments de l'étape (a) du procédé de l'invention peuvent également être générés par des réactions d'amplification comme la PCR menées sur les séquences polynucléotidiques de la banque. Deux solutions sont envisageables. Dans un premier cas, les oligonucléotides amorces peuvent être conçus de manière à générer des fragments dont les extrémités sont adjacentes tout au long de la séquence d'assemblage. Dans un deuxième cas, les oligonucléotides amorces sont conçus de façon à générer des fragments ayant des séquences communes, ces fragments pouvant servir de matrice d'assemblage les uns pour les autres à l'étape (b) ou à l'étape (c). Le procédé de l'invention permet de combiner de manière aléatoire différents fragments obtenus à l'étape (a) et de les réassembler au cours des étapes (b), (c) et (d) au sein d'une séquence polynucléotidique. Ce procédé reproduit donc *in vitro* les phénomènes de recombinaison qui peuvent se produire *in vivo* en les favorisant. Le procédé de l'invention est donc tout particulièrement intéressant pour recombiner des séquences polynucléotidiques entre elles afin de générer de nouvelles séquences polynucléotidiques ayant des propriétés intéressantes par rapport aux propriétés correspondantes de séquences de référence.

L'efficacité de recombinaison du procédé de l'invention est fonction du nombre de fragments générés par séquence polynucléotidique à l'étape (a). En conséquence, le procédé de l'invention utilisera des séquences polynucléotidiques ayant été fragmentés en n fragments, n étant avantageusement supérieur ou égal à trois.

La matrice d'assemblage à l'étape (b) ou (c) est par exemple une séquence polynucléotidique issue de la banque initiale ou une séquence consensus de ladite banque, simple ou double brin. Dans le cas où la matrice d'assemblage est incorporée directement à l'étape (c) de l'invention, cette matrice doit être sous forme simple brin.

Selon une variante du procédé de l'invention, les matrices d'assemblage des étapes (b) ou (c) sont constituées d'oligonucléotides simples ou doubles brins.

Selon une forme particulière de mise en oeuvre du procédé de l'invention, des oligonucléotides, simple ou double brin, de longueur variable, sont ajoutés à l'étape (b) ou (c) en plus de la matrice. Ces oligonucléotides sont conçus pour pouvoir se substituer à une partie des fragments à l'étape (c), en effet, leur séquence est telle que :

- s'ils sont parfaitement homologues avec la séquence du fragment qu'ils remplacent, ils favorisent certaines combinaisons, ou
- s'ils sont partiellement hétérologues avec la séquence du fragment qu'ils remplacent, ils introduisent une ou des mutations dirigées supplémentaires.

Avant l'étape (e) du procédé de l'invention, il est possible de séparer les séquences polynucléotidiques recombinées de la matrice d'assemblage grâce à un marqueur présent sur la matrice d'assemblage ou sur les séquences polynucléotidiques recombinées. Il est en effet possible de marquer chaque brin de la matrice selon des techniques connues de l'homme du métier. Par exemple, le marqueur de la matrice d'assemblage peut être un haptène et l'on sépare les séquences polynucléotidiques recombinées de la matrice d'assemblage par des techniques connues de l'homme du métier, comme par exemple un anticorps anti-haptène fixé sur un support ou une réaction biotine-streptavidine, si l'haptène est un marqueur biotine.

D'autres techniques peuvent être employées pour séparer les séquences polynucléotidiques recombinées de la matrice d'assemblage. La matrice d'assemblage peut aussi être préparée spécifiquement de façon à faciliter son élimination à la fin du procédé de l'invention. Elle peut ainsi être synthétisée par amplification PCR utilisant des dATP méthylés, ce qui permet sa dégradation par l'endonucléase de restriction *Dpn* I. Dans ce cas, les séquences polynucléotidiques recombinées ne doivent pas contenir de dATP méthylés. La matrice peut aussi avoir été préparée par amplification PCR en utilisant des dUTP, ce qui permet sa dégradation par traitement avec une uracyl-DNA-glycosylase. A l'inverse, il est possible de protéger les séquences polynucléotidiques recombinées en les amplifiant par PCR sélective avec des oligonucléotides portant des groupements phosphorothioates en 5'. Un traitement avec une exonucléase permet alors de dégrader spécifiquement la matrice d'assemblage.

Le procédé de l'invention peut comprendre avant le clonage éventuel de l'étape (e), une étape d'amplification des séquences polynucléotidiques recombinées. Toute technique d'amplification est acceptable notamment une amplification par PCR. Une des plus simple consiste à réaliser une PCR qui permet d'amplifier spécifiquement les séquences polynucléotidiques recombinées grâce à des amorces qui ne peuvent s'hybrider que sur les extrémités des séquences recombinées. Les produits PCR sont ensuite clonés pour être caractérisés et les séquences polynucléotidiques présentant des propriétés avantageuses par rapport aux propriétés correspondantes de séquences de référence sont sélectionnées.

L'invention a pour objet de générer des séquences polynucléotidiques susceptibles de présenter des propriétés avantageuses par rapport aux propriétés correspondantes de séquences de référence. Les séquences polynucléotides recombinées obtenues à l'étape (d) et éventuellement clonées sont criblées par tout moyen approprié pour sélectionner les séquences polynucléotidiques recombinées ou les clones présentant des propriétés avantageuses par rapport aux propriétés correspondantes de séquences de référence. On entend, par exemple, par propriétés avantageuses la thermostabilité d'une enzyme ou sa qualité à pouvoir fonctionner dans des conditions de pH ou de température ou de concentration saline plus adaptées à un procédé enzymatique, que les protéines témoins habituellement utilisées pour ledit procédé. A titre d'exemple d'un tel procédé, on peut citer un procédé industriel de désencollage des fibres textiles ou de blanchiment des pâtes à papier ou de la production d'arômes dans l'industrie laitière, les procédés de biocatalyse pour la synthèse par voie enzymatique de nouvelles molécules thérapeutiques, etc...

Selon un mode de réalisation avantageux du procédé de l'invention, la banque de séquence polynucléotidique peut donc être le résultat d'un crible ayant permis de sélectionner par tout moyen approprié les séquences polynucléotidiques présentant des propriétés avantageuses par rapport à des séquences témoins. Les séquences ainsi sélectionnées constituent une banque restreinte.

Mais, il est aussi possible de partir d'une banque non restreinte afin de conserver la représentativité des propriétés contenues dans cette banque.

Les séquences codant pour la ou les protéines présentant une ou des propriétés avantageuses par rapport aux protéines de référence sont alors sélectionnées, par des criblages *in vivo* ou *in vitro,* et peuvent servir à constituer une nouvelle banque pour une éventuelle réitération du procédé de l'invention. Une mise en oeuvre avantageuse du procédé de l'invention consiste donc à utiliser comme banque plusieurs séquences polynucléotidiques sélectionnées après une première mise en oeuvre du procédé de l'invention, éventuellement mélangées avec d'autres séquences polynucléotidiques. Parmi les techniques de criblage qui peuvent être appliquées à chacun des clones de l'étape (e), les techniques de criblage *in vitro* présentent l'avantage de s'affranchir des problèmes de physiologie cellulaire, et de tous les inconvénients liés au clonage d'expression *in vivo*. En outre, ce type de criblage est facilement automatisable, ce qui permet de cribler un nombre élevé de séquences polynucléotidiques recombinées.

L'invention concerne aussi une séquence polynucléotidique recombinée obtenue par un procédé selon l'invention, ainsi qu'un vecteur contenant une telle séquence polynucléotidique recombinée, un hôte cellulaire transformé par une séquence polynucléotidique recombinée ou un vecteur de l'invention, ainsi qu'une protéine codée par cette séquence polynucléotidique recombinée. L'invention comprend également les banques correspondantes de séquences polynucléotidiques recombinées, de vecteurs, d'hôtes cellulaires ou de protéines.

D'autres avantages et caractéristiques de l'invention apparaîtront des exemples de réalisation de l'invention qui suivent et qui se réfèrent aux dessins en annexe dans lesquels :

La figure 1 est une représentation schématique des procédés de l'art antérieur correspondant respectivement au DNA-shuffling (figure 1A) et au StEP figure 1B).

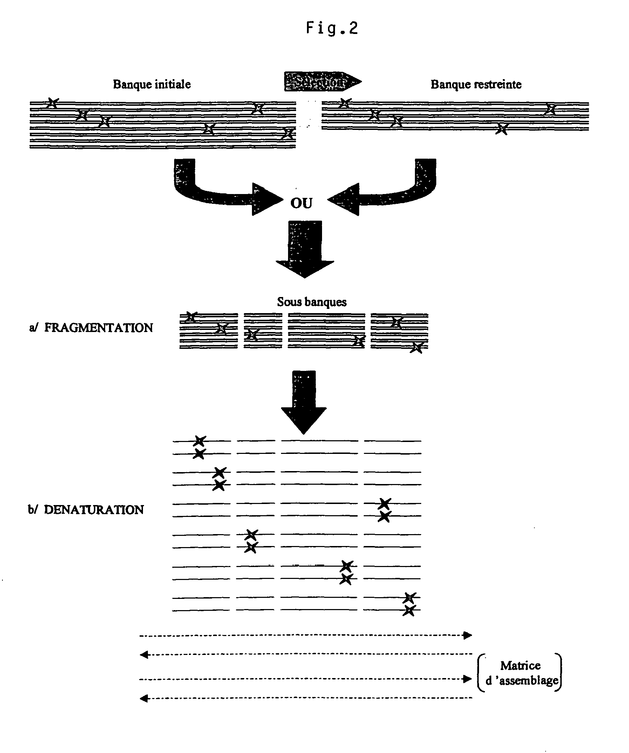

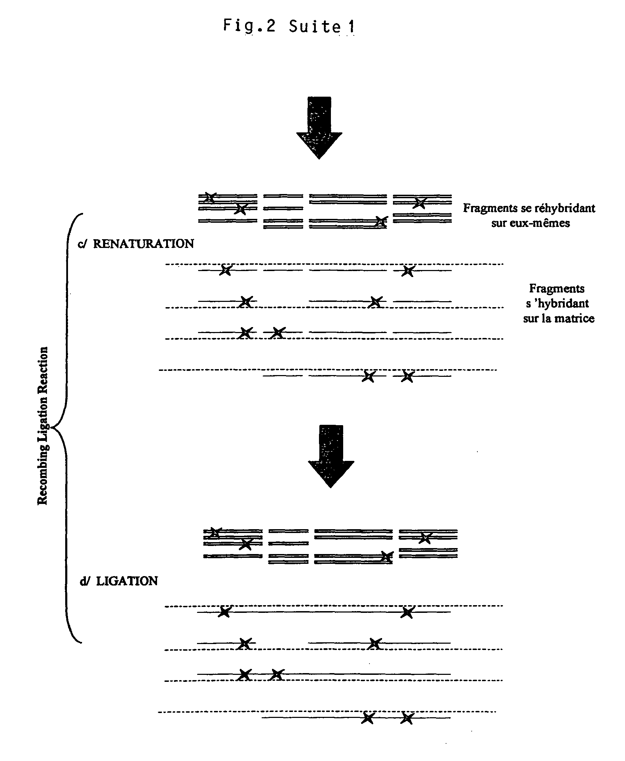

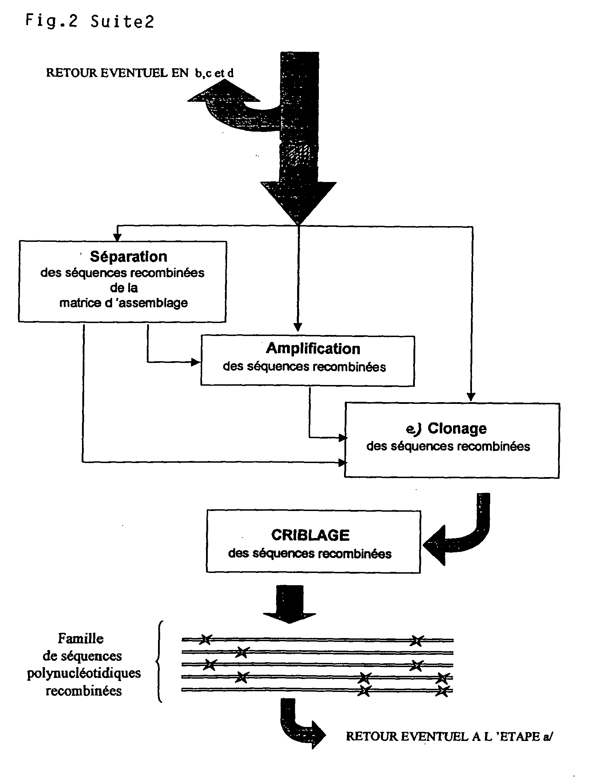

La figure 2 est une représentation schématique d'un exemple de réalisation du procédé de l'invention et de certaines de ses variantes et applications.

La figure 3 représente les positions des dix zones de mutations (*Pvu* II et *Pst* I) portées par chaque mutant du gène *ponB* utilisé pour les exemples de mise en oeuvre de l'invention.

La figure 4 représente la position des amorces utilisées par rapport à la séquence du gène *ponB.*

La figure 5 représente la migration des produits des réactions de RLR et de PCR des produits de ces réactions de RLR sur gel d'agarose.

La figure 6 représente la position des mutations par rapport aux fragments de restriction.

## I - EXEMPLE.

Le procédé de l'invention a été mis en oeuvre à partir d'une banque de mutants du gène *ponB*, codant pour la PBPlb d'*E. coli* (1). Dix mutants de ce gène ont été utilisés. La séquence du gène de chaque mutant diffère de celle du gène sauvage par une zone non homologue de treize à seize bases de long constituée par la substitution de cinq codons initiaux par cinq codons alanines selon la technique décrite par Lefèvre *et al.* (8).

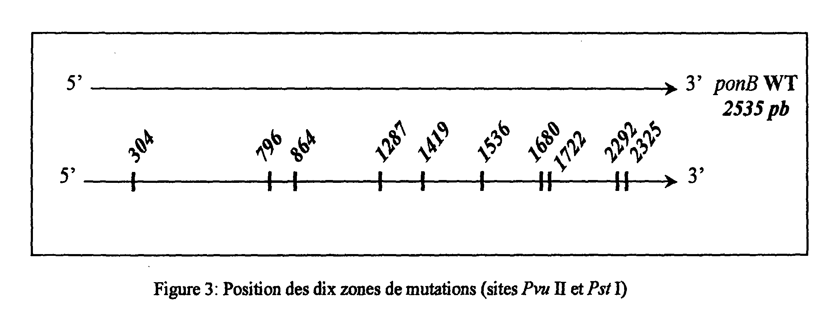

La substitution portée par chaque mutant est caractérisée par la présence d'un site unique de l'enzyme de restriction *Pvu* II encadré par deux sites de l'enzyme *Pst* I, qui permettent de distinguer les mutants les uns des autres par leur profil de digestion avec ces endonucléases de restriction. La figure 3 représente les positions des dix zones de mutations (*Pvu* II et *Pst* I) portées par chaque mutant.

Après amplification par PCR des gènes des dix mutants, les produits PCR ont été purifiés, mélangés en quantité équimolaire pour constituer la banque. Les séquences polynucléotidiques de cette banque ont été digérées par les enzymes de restriction *Hinf* I et *Bsa* I, de façon à générer des banques de fragments de restriction. Les fragments de restriction ont alors été incubés avec la matrice sauvage, à différentes quantités, en présence d'une ligase thermostable. Après plusieurs cycles de dénaturation/hybridation/ligation, une fraction de ce mélange réactionnel a été utilisée pour réaliser une amplification PCR avec un couple d'amorces spécifiques des extrémités 5' et 3' des gènes des mutants et non spécifiques des extrémités 5' et 3' de la matrice_sauvage. Le produit d'amplification a été cloné, et les clones obtenus ont été analysés pour leur profil de digestion avec l'endonucléase de restriction *Pvu* II ou *Pst* I. Les profils obtenus ont permis de déterminer quel(s) fragment(s) des mutants avai(en)t pu se recombiner avec les autres pour reconstituer un gène entier.

## II - MATERIEL.

1) Souches et plasmides.

La souche MC1061 (F$^-$ *araD*139, $\Delta$(*ara-leu*)$_{7696}$, *galE15, galK16*, $\Delta$(*lac*)*X*74, *rpsL* (Str$^R$), *mcrA mcrB1* , *hsdR2* ($r_k^-$ $m_k^+$)) est dérivée d'*Escherichia coli* K12.

Le vecteur pARAPONB est issu du vecteur pARA13 (3) dans lequel le gène *ponB* portant un site de coupure thrombine (9) a été introduit entre les sites de restriction *Nco* I et *Nar* I. Le vecteur pET26b+ fait partie de la famille des vecteurs pET développée par Studier et Moffatt (13) et commercialisés par la société NOVAGEN.

2) Oligonucléotides.

Les oligonucléotides ont été synthétisés par la société ISOPRIM (Toulouse). Les séquences des oligonucléotides sont rapportées dans le tableau I ci-dessous.

## Tableau I

| Oligo N | 5' ACTGACTACCATGGCCGGGAATGACCGCGAGCC 3' |
| Oligo E | 5' CCGCGGTGGAGCGAATTCTAATTACTACCAAACATATCC 3' |
| Oligo M1 | 5' GCGCCTGAATATTGCGGAGAAAAAGC 3' |
| Oligo M2 | 5' ACAACCAGATGAAAAGAAAGGGTTAATATC 3' |
| Oligo A1 | 5' ACTGACTACCATGGCC 3' |
| Oligo A2 | 5' CCGCGGTGGAGCGAATTC 3' |

3) Réactifs.
Les enzymes de restriction et de modifications citées dans le tableau II ci-dessous ont été utilisées selon les recommandations des fournisseurs.

Tableau II

| Enzyme | Concentration | Fournisseur |
| --- | --- | --- |
| *Nco*I | 10 U/μl | NEB |
| *Pst*I | 20 U/μl | NEB |
| *Eco R*I | 20 U/μl | NEB |
| *Bsa* I | 5 U/μl | New England Biolabs |
| *Hinf* I | 10 U/μl | New England Biolabs |
| *Pvu* II | 10 U/μl | New England Biolabs |
| T4 DNA ligase | 400 U/μl | New England Biolabs |
| Taq ADN polymérase | 5 U/μl | PROMEGA |
| AMPLIGASE | 100 U/μl | EPICENTRE |

Les tampons utilisés sont raportés dans le tableau III ci-dessous.

Tableau III

| Tampons | Composition |
| --- | --- |
| T | Tris HCl 10 mM, pH 8.0 |
| Polymérisation 20X | Tris HCl 100 mM pH 8,3, MgCl$_2$ 15 mM, KCl 500 mM, 1.0% Triton X100® |
| Restriction A 10X | 500 mM NaCl, 100 mM Tris HCl pH 7,9, 100 mM MgCl$_2$, 10 mM DTT |
| Restriction B 10X | 1 M NaCl, 500 mM Tris HCl pH 7,9, 100 mM MgCl$_2$, 10 mM DTT |
| Restriction C 10X | 500 mM NaCl, 1 M Tris HCl pH 7,5, 100 mM MgCl$_2$, 0,25% Triton X100® |
| AMPLIGASE 10X | 200 mM Tris HCl pH 8,3, 250 mM KCl, 100 mM MgCl2, 5 mM NAD, 0,1% Triton X100® |
| Ligation 10X | 500 mM Tris HCl pH 7,5, 100 mM MgCl$_2$, 100 mM DTT, 10 mM ATP, 250 μg/ml BSA |

III - PREPARATION DE LA MATRICE.

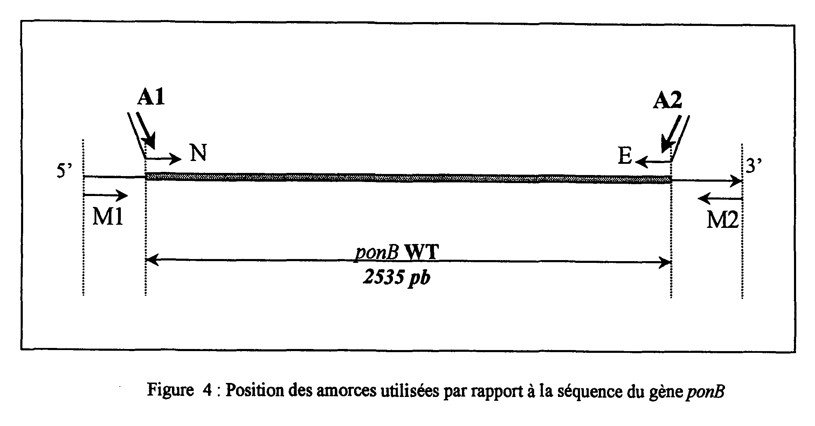

Le gène *ponB* sauvage a été amplifié par une étape de réaction de PCR en utilisant comme amorces les oligonucléotides M1 et M2(fig. 4). Cinq réactions de PCR ont été préparées en ajoutant 50 ng du plasmide pPONBPBR portant le gène sauvage (7) à un mélange contenant 10 μl de tampon de polymérisation, 10 μl de dNTPs 2mM, 20 pmol de chaque oligonucléotide M1 et M2, et 5U de Taq ADN polymérase, dans un volume final de 100 μl. Ces mélanges

ont été incubés dans un thermocycleur Perkin-Elmer 9600 selon le programme suivant : (94°C - 2 min.) - ( 94°C 15 sec. - 60°C 30 sec. - 72°C 1 min.) x 29 cycles - (72°C - 3 min.).

Le produit des cinq PCR ont été mélangés et déposés sur un gel d'agarose TBE 1%. Après migration et coloration au bromure d'éthidium du gel, la bande à 2651 pb, correspondant au produit d'amplification du gène *ponB* encadré par deux fragments de 26 pb et 90 pb respectivement, a été visualisée par trans-illumination aux ultraviolets, et découpée à l'aide d'un scalpel pour être purifiée avec le système Quiaquick (QIAGEN). La totalité de l'ADN ainsi purifié a été éluée dans 120 µl de tampon T. La concentration de cet ADN a été évaluée par un dosage spectrophotométrique à 260 nm, à 100 ng/µl.

IV - PREPARATION DE LA BANQUE.

1) Amplification des gènes mutants.

Les gènes des dix mutants ont été amplifiés séparément par l'intermédiaire d'une réaction de PCR en utilisant les oligonucléotides N et E. Ces oligonucléotides introduisent respectivement les sites de restriction *Nco* I et *Eco* RI, permettant de cloner les produits obtenus avec ces deux sites.

Chaque réaction de PCR a été préparée en ajoutant 50 ng du plasmide portant le gène mutant à un mélange contenant 10 µl de tampon de polymérisation, 10 µl de dNTPs 2mM, 20 pmol de chaque oligonucléotide N et E, et 5U de Taq ADN polymérase, dans un volume final de 100 µl. Ce mélange a été incubé dans un thermocycleur Perkin-Elmer 9600 selon le programme suivant : (94°C - 2 min.) - ( 94°C 15 sec. - 60°C 30 sec. - 72°C 1 min.) x 29 cycles - (72°C - 3 min.).

La spécificité de l'amplification génique a été vérifiée par profil de restriction avec l'endonucléase *Pvu* II, en incubant 5 µl de chaque produit PCR 1 heure à 37°C dans un mélange contenant 3 µl de tampon de restriction A et 5U de l'enzyme *Pvu* II dans un volume final de 30 µl. 15 µl de cette réaction de digestion ont été déposés sur un gel d'agarose TBE 1%. Après migration et coloration au bromure d'éthidium, le gel a été exposé aux ultraviolets. La visualisation des fragments de restriction a permis de confirmer la spécificité de l'amplification génique de chaque gène mutant.

En parallèle, 3 µl de chaque réaction PCR ont été déposés sur un gel d'agarose TBE 1%. Après migration, le gel a été traité comme ci-dessus. L'intensité de chaque bande a permis d'estimer que les amplifications géniques avaient eu le même rendement.

2) Création des banques de fragments de restriction.

50 µl de chacune des dix PCR ont été mélangés et déposés sur un gel d'agarose TBE 1%. Après migration et coloration au bromure d'éthidium, la bande à 2572 pb, correspondant au produit d'amplification des gènes des dix mutants, a été découpée à l'aide d'un scalpel et purifiée avec le système Quiaquick (QIAGEN). La totalité de l'ADN ainsi purifié a été éluée dans 120 µl de tampon T. La concentration de cet ADN a été évaluée par un dosage spectrophotométrique à 260 nm, à 100 ng/µl.

Pour générer les banques de fragments de restriction, 100 µl de cet ADN ont été incubés une heure à 50°C dans un mélange contenant 12 µl de tampon de restriction B, 1,2 µl de BSA (à 10 mg/ml), 25 U de l'enzyme *Bsa* I et 4 µl d'eau. Puis 2 µl du tampon de restriction B, 2 µl de BSA (à 1mg/ml), 50 U de l'enzyme *Hinf* I et 11,5 µl d'eau ont été rajoutés au mélange, qui a été incubé une heure à 37°C. La totalité du mélange de digestion a été purifié sur une colonne QIAquick (QIAGEN), et élué avec 30 µl de tampon T. 1 µl de cet éluat a été déposé sur gel d'agarose TBE 1% pour vérifier que la digestion avait été totale, et qu'elle avait généré 6 fragments de restriction, et par conséquent six banques de fragments, de 590 pb, 500 pb, 472 pb, 438 pb, 298 pb et 274 pb. La concentration de cet ADN a été évaluée (par un dosage spectrophotométrique à 260 nm) à 250 ng/µl.

V - RLR (Recombinating Ligation Reaction).

La réaction de RLR (Recombining Ligation Reaction) a été réalisée en incubant des quantités déterminées de fragments de restriction *Hinf* I - *Bsa* I des gènes des dix mutants avec la matrice complète (*ie* le gène *ponB* sauvage), en présence d'une ADN ligase thermostable. Le tableau IV ci-dessous rapporte la composition des mélanges de RLR.

Tableau IV

|  | RLR 1 | RLR 2 | RLR3 | RLR4 | T- |
|---|---|---|---|---|---|
| Fragments *Hinf* I - *Bsa* I des dix mutants (100 ng/µl) | 0,5 µl | 1 µl | 2 µl | 5 µl | 5 µl |
| Matrice *ponB* sauvage (100 ng/µl) | 0,6 µl | 1,2 µl | 2,4 µl | 6 µl | 6 µl |

(suite)

|  | RLR 1 | RLR 2 | RLR3 | RLR4 | T- |
|---|---|---|---|---|---|
| Tampon AMPLIGASE 10X | 2 $\mu$l | 2 $\mu$l | 2 $\mu$l | 2 $\mu$l | 2 $\mu$l |
| AMPLIGASE (25 U/$\mu$l) | 1 $\mu$l | 1 $\mu$l | 1 $\mu$l | 1 $\mu$l | - |
| H$_2$O | qsp 20 $\mu$l | qsp 20 $\mu$l | qsp 20 $\mu$l | qsp 20 $\mu$l | qsp 20 $\mu$l |

Le témoin négatif est identique à la réaction de RLR4, mais ne contient pas d'ADN ligase thermostable. Ces différents mélanges ont été recouverts d'une goutte d'huile minérale et incubés dans un thermocycleur Perkin-Elmer 9600 dans des microtubes de 200 $\mu$l selon le programme suivant : (94°C 5 min.) - (94°C 1 min. - 65°C 4 min.) x 35 cycles.

10 $\mu$l de chaque réaction de RLR ont alors été ajoutés à un mélange de réaction PCR contenant 10 $\mu$l de tampon de polymérisation, 10 $\mu$l de dNTPs 2 mM, 40 pmol de chaque oligonucléotide A1 et A2, et 5 U de Taq ADN polymérase dans un volume final de 100 $\mu$l. Ce mélange a été incubé dans un thermocycleur Perkin-Elmer 9600 selon le programme suivant : (94°C 5 min. ) - (94°C 30 sec. - 46°C 30 sec. - 72°C 1 min.) x 29 cycles - (72°C 2 min.). Cette réaction de PCR a permis d'amplifier spécifiquement les produits de ligation formés au cours de la réaction de RLR, sans amplifier la matrice, puisque les oligonucléotides A1 et A2 ne peuvent pas s'hybrider sur celle-ci, comme montré sur la figure 4.

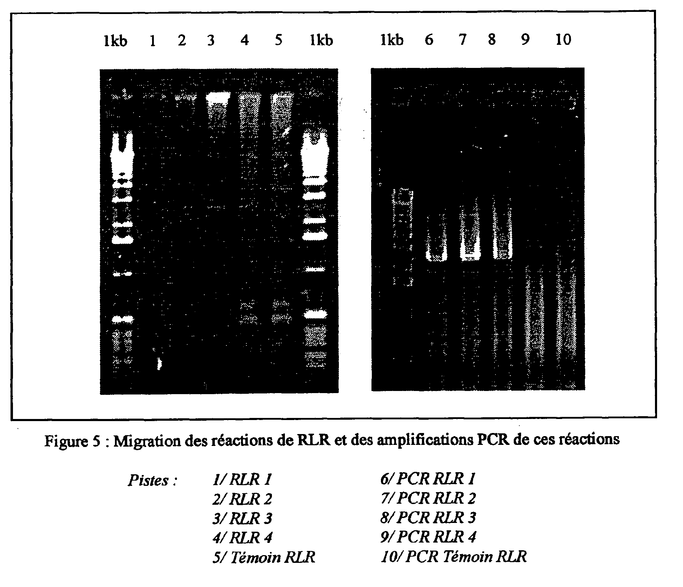

5 $\mu$l de chaque réaction de RLR et 10 $\mu$l de chacune des réactions de PCR précédentes ont été déposés sur un gel d'agarose TBE 1%. Après coloration au bromure d'éthidium, le gel a été exposé aux ultraviolets, comme montré à la figure 5.

L'analyse de ce gel révèle que seule la réaction de RLR4 contient, comme le témoin négatif, des fragments de restrictions encore visibles (pistes 4 et 5).

L'absence de produit PCR pour le témoin négatif (piste 10) révèle non seulement que la réaction PCR est spécifique (pas d'amplification de la matrice complète), mais aussi que les fragments de restriction présents dans le mélange ne peuvent pas se substituer aux amorces pour générer un produit PCR contaminant dans les conditions choisies. En parallèle, la présence d'une bande unique à environ 2500 pb sur les pistes 6, 7 et 8 démontre qu'un produit de RLR a pu être amplifié par PCR pour les réactions de RLR1, 2 et 3. Ces trois réactions de RLR ont donc permis de reconstituer un ou des gènes complets à partir de six banques de fragments de restrictions.

VI - ANALYSE DES PRODUITS D'AMPLIFICATION DES REACTIONS DE RLR.

1) Clonage.

Les produits d'amplification PCR des réactions de RLR 1, 2 et 3 ont été purifiés avec le système Wizard PCR Preps (PROMEGA) et élues dans 45 $\mu$l de tampon T. 6 $\mu$l de chaque PCR purifiée ont été incubés 1 heure à 37°C dans un mélange contenant 3 $\mu$l de tampon de restriction C, 3 $\mu$l de BSA (à lmg/ml), 20 U de l'enzyme *Eco* RI, 10 U de l'enzyme *Nco* I et 15 $\mu$l d'eau.

En parallèle, deux vecteurs (pARAPONB et pET26b+) ont été préparés pour le clonage. Ces vecteurs ont été linéarisés en incubant 3 $\mu$g de ces plasmides 2 heures à 37°C, dans un mélange contenant 3 $\mu$l de tampon de restriction C, 3 $\mu$l de BSA (à lmg/ml), 20 U de l'enzyme *Eco* RI, 10 U de l'enzyme *Nco* I et 19 $\mu$l d'eau.

Les vecteurs linéarisés ainsi que les PCR digérées ont été purifiés sur gel d'agarose TBE 1% avec le système QIAquick (QUIAGEN). Chaque vecteur ou chaque PCR digérée a été éluée dans 30 $\mu$l de tampon T.

La ligation de chaque PCR digérée avec l'un ou l'autre des vecteurs a été réalisée selon les conditions décrites dans le tableau V ci-dessous, et incubée à 16°C pendant 16 heures.

Tableau V

|  | Ligation avec le vecteur pARAPONB | | | | Ligation avec le vecteur pET26b+ | | | |
|---|---|---|---|---|---|---|---|---|
|  | LpAR1 | LpAR2 | LpAR3 | TLpAR | LpET1 | LpET2 | LpET3 | TLpET |
| PCR amplification RLR 1 digérée *Nco* I - *Eco* RI | 4 $\mu$l | - | - | - | 4 $\mu$l | - | - | - |
| PCR amplification RLR 2 digérée *Nco* I - *Eco* RI | - | 4 $\mu$l | - | - | - | 4 $\mu$l | - | - |
| PCR amplification RLR 3 digérée *Nco* I - *Eco* RI | - | - | 4 $\mu$l | - | - | - | 4 $\mu$l | - |

(suite)

| | Ligation avec le vecteur pARAPONB | | | | Ligation avec le vecteur pET26b+ | | | |
|---|---|---|---|---|---|---|---|---|
| | LpAR1 | LpAR2 | LpAR3 | TLpAR | LpET1 | LpET2 | LpET3 | TLpET |
| Vecteur pARAPONB digérée *Nco* I - *Eco* RI | 1 μl | 1 μl | 1 μl | 1 μl | - | - | - | - |
| Vecteur pET26b+ digérée *Nco* I - *Eco* RI | - | - | - | - | 1 μl | 1 μl | 1 μl | 1 μl |
| Tampon de ligation | 2 μl | 2 μl | 2 μl | 2 μl | 2 μl | 2 μl | 2 μl | 2 μl |
| Ligase | 1 μl | 1 μl | 1 μl | 1 μl | 1 μl | 1 μl | 1 μl | 1 μl |
| H$_2$O | 12 μl | 12 μl | 12 μl | 16 μl | 12 μl | 12 μl | 12 μl | 16 μl |

200 μl de cellules MC1061 chimiocompétentes (4) ont été transformées avec 10 μl de chaque ligation par un choc thermique (5), et les cellules ainsi transformées ont été étalés sur un milieu de sélection.

Aucun clone n'a été obtenu après transformation des témoins de ligation TL pAR et TL pET, indiquant ainsi que les vecteurs pARAPONB et pET26b+ linéarisés *Nco* I - *Eco* RI ne peuvent pas subir une ligation intramoléculaire.

2) Criblage par PCR.

Un premier criblage des clones obtenus après transformation des ligations avec le vecteur pARAPONB a été réalisée par PCR. 42 colonies, 14 de chaque ligation LpAR1, LpAR2 et LpAR3, ont été resuspendues individuellement dans un mélange de PCR contenant 5 μl de tampon de polymérisation, 40 pmol de chaque oligonucléotide A1 et A2, 5 μl de dNTPs 2mM et 5U de Taq ADN polymérase dans un volume final de 50 μl. Un témoin négatif a été constitué en ajoutant au mélange de PCR 50 ng du plasmide pBR322 à la place d'une colonie. Ces 43 tubes ont été incubés dans un thermocycleur Perkin-Elmer 9600 selon le programme suivant : (94°C 5 min.) - (94°C 30 sec. - 46°C 30 sec. - 72°C 1 min.) x 29 cycles - (72°C 2 min.). 5 μl de chacune de ces réactions PCR ont ensuite été incubés 1 heure à 37°C dans un mélange contenant 2 μl de tampon de restriction A, 2 μl de BSA (à 1 mg/ml) et 5 U de l'enzyme de restriction *Pvu* II dans un volume final de 20 μl.

10 μl de chacune de ces digestions ont été déposées sur un gel d'agarose TBE 1% en parallèle de 5 μl de chaque PCR non digérée (ce qui permet de ne pas confondre d'éventuelles bandes non spécifiques de la PCR avec un fragment obtenu par digestion de restriction). Après migration et coloration au bromure d'éthidium de ce gel, les bandes issues de la digestion par l'enzyme *Pvu* II ont été analysées afin de déterminer quel(s) fragment(s) des mutants initiaux s'étaient associé(s) avec les autres pour reconstituer un gène entier. Ce criblage révèle la présence de 27 gènes portant une mutation, 7 gènes portant deux mutations et de 8 gènes ne portant plus de mutation.

3) Criblage par minipréparation d'ADN plasmidique.

Le deuxième criblage a été effectué en réalisant une extraction de l'ADN plasmidique (5) de 21 clones issus de la transformation des ligations avec le vecteur pET26b+ (7 clones de chaque ligation). 5 μl de l'ADN plasmidique ainsi obtenu pour chaque clone a été incubé 1 heure à 37°C en présence d'un mélange contenant 1 μl de tampon de restriction C, 6 U de l'enzyme *Pst* I, 3 U de l'enzyme *Nco* I et 6 U de l'enzyme *Eco* RI dans un volume final de 10 μl. 5 μl de chacune de ces digestions ont été déposées sur un gel d'agarose TBE 1%. Après migration et coloration au bromure d'éthidium de ce gel, les bandes issues de la digestion par l'enzyme *Pst* I ont été analysées afin de déterminer quel(s) fragment(s) des mutants initiaux s'étaient associé(s) avec les autres pour reconstituer un gène entier. Ce criblage révèle la présence de 13 gènes portant une mutation, 5 gènes portant deux mutations et de 3 gènes ne portant plus de mutation.

4) Analyse statistique des recombinaisons.

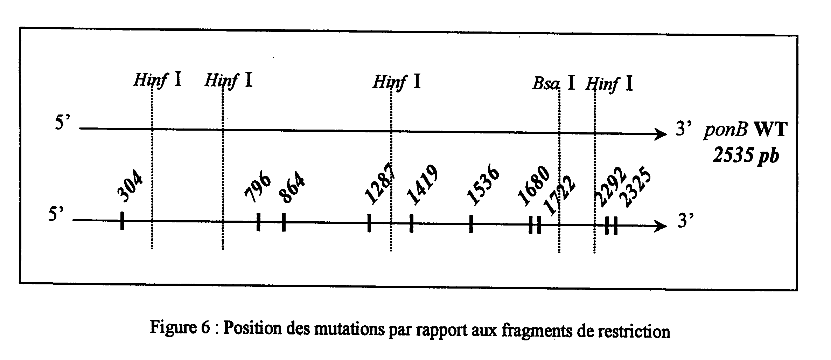

En fonction de la position de chaque mutation par rapport aux sites de coupure des enzymes *Hinf* I et *Bsa* I, comme représenté à la figure 6, il est possible de calculer la probabilité d'obtenir au cours de la réaction de RLR la création d'un gène portant 0, 1, 2, 3 ou 4 des mutations des gènes initiaux.

Ainsi, en considérant que la réaction de RLR est totalement aléatoire les probabilités P sont les suivantes :

$$P(0 \text{ mutation}) = \prod_{i=6}^{9}\left(\frac{i}{10}\right) = 30,24\%$$

$$P(1 \text{ mutation}) \quad = \sum_{n=1}^{4}\left[\frac{n}{10-n}\prod_{i=1}^{4}\left(\frac{10-i}{10}\right)\right] = 44,04\%$$

$$P(2 \text{ mutations}) \quad = \sum_{n=1}^{4}\left[\sum_{a=1}^{4-n}\left(\frac{10-a}{a}\right)\left(\frac{10-(a+n)}{a+n}\right)\prod_{i=1}^{4}\left(\frac{i}{10}\right)\right] = 21,44\%$$

$$P(3 \text{ mutations}) \quad = \sum_{n=1}^{4}\left[\left(\frac{10-n}{n}\right)\prod_{i=1}^{4}\left(\frac{i}{10}\right)\right] = 4,04\%$$

$$P(4 \text{ mutations}) \quad = \prod_{i=1}^{4}\left(\frac{i}{10}\right) = 0,24\%$$

Les deux criblages effectués donnent des résultats proches de ces prévisions statistiques, comme rapporté dans le tableau VI ci-dessous, indiquant ainsi que la réaction de RLR est quasi aléatoire. Une proportion légèrement plus élevée de gènes portant une mutation, au détriment des gènes portant zéro mutation est observée. Ce phénomène pourrait être attribué à une faible toxicité du gène *ponB* déjà observée et à la légère fuite des vecteurs d'expression pARAPONB et pET26b+, qui favoriseraient la sélection de gènes portant une mutation inactivante.

Tableau VI

| % | 0 mutation | 1 mutation | 2 mutations | 3 mutations | 4 mutations |
|---|---|---|---|---|---|
| Statistique | 30,24 | 44,04 | 21,44 | 4,04 | 0,24 |
| Criblage PCR | 21 | 63 | 16 | 0 | 0 |
| Criblage mini-préparation | 14 | 62 | 24 | 0 | 0 |

RÉFÉRENCES BIBLIOGRAPHIQUES

1) Broome-Smith J.K., Edelman A., Yousif S.et Spratt B.G., (1985), The nucleotide sequence of the *ponA* and *ponB* genes encoding penicillin-binding proteins 1A and 1B of *Escherichia coli* K12, Eur. J. Biochem., **147**, 437-446.

2) Cadwell R.C. et Joyce G., 1992, Randomization of genes by PCR mutagenesis, PCR Methods and Application, **2**, 28-33.

3) Cagnon C., Valaverde V. et Masson J.-M., (1991), A new family of sugar inductible expression vectors for *Escherichia coli,* Prot. Eng., **4**, 843-847.

4) Hanahan D., (1985), Techniques for transformation of *Escherichia coli*, dans DNA cloning : a practical approach, Glover D.M. (Ed), IRL Press, Oxford **vol I**, 109-135.

5) Maniatis T., Fristch E.F. et Sambrook J., (1982), Molecular cloning. A laboratory Manual, Cold Spring Harbor Laboratory, Cold Spring Harbor, NY.

6) Landt et al., Gene, 96, 125-128, 1990

7) Lefèvre F., Analyse topologique de la Penicillin Binding Protein 1b d'*Escherichia coli,* 1997, Thèse.

8) Lefèvre F., Rémy M.H. et Masson J.M., 1997 (a), Alanine-stretch scanning mutagenesis : a simple and efficient method to probe protein structure and function, Nuc. Acids Res., **25**, 447-448.

9) Lefèvre F., Rémy M.H. et Masson J.M., 1997 (b), Topographical and functional investigation of *Escherichia coli* Penicillin-Binding Protein 1b by alanine stretch scanning mutagenesis, J. Bacteriol., **179**, 4761-4767.

10) Lyamichev V., Mast A.L., Prudent J.R., Kaiser M.W., Takova T., Kwiatkowski R.W., Sander T.J., de Arruda M., Arco D.A., Neri B.P. et Brown M.A.D., 1999, Polymorphism identification and quantitative detection of genomic DNA by invasive cleavage of oligonucleotide probes, Nature Biotechnology, **17,** 292-296.

11) Picard et al., Nuc. Acids Res., 22, 2587-2591, 1994.

12) Stemmer W.P.C., (1994), Rapid evolution of a protein *in vitro* by DNA shuffling, Nature, **370**, 141-144.

13) Studier F.W. et Moffatt B.A., 1986, Use of bacteriophage T7 RNA polymerase to direct selective high-level expression of cloned genes, J. Mol. Biol, **189,** 113-130.

14) Zhao H., Giver L., Shao Z., Affholter J.A. et Arnold F., 1998, Molecular evolution by staggered extension process (StEP) in vitro recombination, Nature Biotech., **16,** 258-261.

<u>Annexe aux documents de la demande - listage des séquences déposé ultérieurement</u>

**Revendications**

**1.** Procédé de préparation *in vitro* d'une banque de polynucléotidiques **caractérisé en ce qu'**il comprend les étapes suivantes :

  a) la fragmentation d'une banque de séquences polynucléotidiques
  b) l'hybridation des fragments obtenus sur une matrice d'assemblage
  c) la ligation des fragments hybridés possédant des extrémités adjacentes à l'aide d'une ligase
  d) la dénaturation des fragments ligués de la matrice d'assemblage ; et la répétition des étapes d'hybridation (b), de ligation (c) et de dénaturation (d) pour former plusieurs recombinants aléatoires,

ledit procédé étant en outre **caractérisé par le fait que** les étapes (a) à (d) sont réalisées en absence de polymérase

2. Procédé de préparation *in vitro* d'une banque de polynucléotidiques selon la revendication 1, **caractérisé en ce qu'**il comprend les étapes suivantes :

a) la fragmentation d'une banque de séquences polynucléotidiques dont l'une au moins présente des propriétés de référence,
b) l'hybridation des fragments obtenus sur une matrice d'assemblage,
c) la ligation des fragments hybridés possédant des extrémités adjacentes à l'aide d'une ligase,
d) la dénaturation des fragments ligués de la matrice d'assemblage ; et la répétition des étapes d'hybridation (b), de ligation (c) et de dénaturation (d) pour former plusieurs recombinants aléatoires, l'un au moins desdits recombinants étant susceptible de posséder des propriétés avantageuses par rapport auxdites propriétés de référence,

ledit procédé étant en outre **caractérisé par le fait que** les étapes (a) à (d) sont réalisées en absence de polymérase.

3. Une banque de polynucléotidiques recombinés susceptible d'être obtenue par le procédé selon l'une des revendications 1 ou 2.

4. Une banque de polynucléotidiques recombinés selon la revendication 3, **caractérisée en ce qu'**elle comprend au moins un recombinant susceptible de posséder des propriétés avantageuses par rapport à des propriétés de référence d'au moins une séquence polynucléotidique de la banque utilisée pour obtenir ladite banque de polynucléotidiques recombinés.

5. Une séquence polynucléotide recombinée sélectionnée par criblage à partir d'une banque de polynucléotidiques recombinés selon l'une des revendications 3 ou 4.

6. Une séquence polynucléotide recombinée selon la revendication 5, **caractérisée en ce qu'**elle est sélectionnée par criblage sur les propriétés de référence d'au moins une séquence polynucléotidique de la banque utilisée pour obtenir la banque de polynucléotidiques recombinés.

7. Une séquence polynucléotidique recombinée selon la revendication 6, **caractérisée en ce qu'**elle code pour une protéine présentant des propriétés améliorées par rapport aux propriétés de référence d'une protéine codée par au moins une séquence polynucléotidique de la banque utilisée pour obtenir la banque de polynucléotidiques recombinés.

8. Une protéine codée par une séquence polynucléotidique recombinée selon l'une quelconque des revendications 5 à 7.

9. Un vecteur contenant une séquence polynucléotidique recombinée selon l'une quelconque des revendications 5 à 7.

10. Un hôte cellulaire transformé par une séquence polynucléotidique recombinée selon l'une quelconque des revendications 5 à 7 ou par un vecteur selon la revendication 9.

Figure 1 A

Famille de gènes

Fragmentation
DNAse I

PCR
sans amorces

PCR
avec amorces

Figure 1 B

(Procédé réalisé en double brin, mais illustré ici sur un seul brin)

Famille de gènes { Gène A

Gène B

Cycles courts de dénaturation, hybridation, polymérisation

Famille de gènes recombinés

Fig.2

Banque initiale        Sélection        Banque restreinte

OU

Sous banques

a/ FRAGMENTATION

b/ DENATURATION

Matrice
d'assemblage

# Fig.2 Suite 1

**c/ RENATURATION**

Fragments se réhybridant sur eux-mêmes

Fragments s'hybridant sur la matrice

**Recombing Ligation Reaction**

**d/ LIGATION**

EP 1 683 861 A1

Fig.2 Suite2

RETOUR EVENTUEL EN b,c et d

**Séparation**
des séquences recombinées
de la
matrice d'assemblage

**Amplification**
des séquences recombinées

**e) Clonage**
des séquences recombinées

**CRIBLAGE**
des séquences recombinées

Famille
de séquences
polynucléotidiques
recombinées

RETOUR EVENTUEL A L'ETAPE a/

22

Figure 3: Position des dix zones de mutations (sites *Pvu* II et *Pst* I)

Figure 4 : Position des amorces utilisées par rapport à la séquence du gène *ponB*

Figure 5 : Migration des réactions de RLR et des amplifications PCR de ces réactions

Pistes :
1/ RLR 1          6/ PCR RLR 1
2/ RLR 2          7/ PCR RLR 2
3/ RLR 3          8/ PCR RLR 3
4/ RLR 4          9/ PCR RLR 4
5/ Témoin RLR     10/ PCR Témoin RLR

Figure 6 : Position des mutations par rapport aux fragments de restriction

**Office européen**

**des brevets**

## RAPPORT PARTIEL
## DE RECHERCHE EUROPEENNE
qui selon la règle 45 de la Convention sur le brevet européen est consideré, aux fins de la procédure ultérieure, comme le rapport de la recherche européenne

Numéro de la demande

EP 05 29 1780

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | STEMMER W P C: "DNA SHUFFLING BY RANDOM FRAGMENTATION AND REASSEMBLY: IN VITRO RECOMBINATION FOR MOLECULAR EVOLUTION" PROCEEDINGS OF THE NATIONAL ACADEMY OF SCIENCES OF USA, vol. 91, 1 octobre 1994 (1994-10-01), pages 10747-10751, XP002912215 ----- | | INV.<br>C12N15/10<br>C12N15/31<br>C12N1/21<br>C07K14/00 |
| A | WO 98/27230 A (MAXYGEN INC ;PATTEN PHILLIP A (US); STEMMER WILLEM P C (US)) 25 juin 1998 (1998-06-25) * page 13, ligne 15 - page 15, ligne 21 * ----- | | |
| A | WO 98/15567 A (KONCZ CSABA ;SCHELL JEFF (DE); STRIZHOV NICOLAI (DE); VITALITY BIO) 16 avril 1998 (1998-04-16) * page 2, dernier alinéa - page 4 * ----- <br><br> -/-- | | |

DOMAINES TECHNIQUES RECHERCHES (IPC)

C12N
C07K

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Munich | 8 juin 2006 | Marinoni, J-C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
............................................................................
& : membre de la même famille, document correspondant

EPO FORM 1503 03.82 (P04C08)

Office européen
des brevets

**RAPPORT PARTIEL
DE RECHERCHE EUROPEENNE**

Numero de la demande

EP 05 29 1780

| | DOCUMENTS CONSIDERES COMME PERTINENTS | | CLASSEMENT DE LA DEMANDE (IPC) |
|---|---|---|---|
| Catégorie | Citation du document avec indication, en cas de besoin, des parties pertinentes | Revendication concernée | |
| A | WEISBERG E P ET AL: "SIMULTANEOUS MUTAGENESIS OF MULTIPLE SITES: APPLICATION OF THE LIGASE CHAIN REACTION USING PCR PRODUCTS INSTEAD OF OLIGONUCLEOTIDES" BIOTECHNIQUES, vol. 15, no. 1, 1 juillet 1993 (1993-07-01), pages 68-70, 72 - 74,, XP000385832 * le document en entier * ----- | | |
| A | WO 98/32845 A (CRIPPS JOANNA E ;SOEDERLIND ULF HANS ESKIL (SE); BIOINVENT INT AB) 30 juillet 1998 (1998-07-30) * le document en entier * ----- | | DOMAINES TECHNIQUES RECHERCHES (IPC) |
| D,A | ZHAO H ET AL: "Molecular evolution by staggerd extension process (StEP) in vitro recombination" NATURE BIOTECHNOLOGY, vol. 16, mars 1998 (1998-03), pages 258-261, XP002104755 * le document en entier * ----- | | |

EPO FORM 1503 03.82 (P04C11)

**Office européen des brevets**

**RECHERCHE INCOMPLETE**
**FEUILLE SUPPLEMENTAIRE C**

Numéro de la demande

EP 05 29 1780

Revendications ayant fait l'objet de recherches complètes:
        1,2

Revendications n'ayant pas fait l'objet de recherches:
        3-10

Raison pour la limitation de la recherche:

Les revendications 3-10 couvrent des produits (séquences polynucléotidiques, protéines codés par ces séquences, vecteurs comprenant ces séquences, cellules contenant ces vecteurs ou ces séquences) définis seulement par le fait que lesdites séquences sont obtenues par la méthode des revendications 1 ou 2, contrairement aux exigences de l'article 84 CBE. Cependant une telle définition du produit par le procédé utilisé pour l'obtenir ne permet pas de déterminer l'étendue de la protection. Le fait que chaque composé puisse être testé ne permet pas de surmonter cette objection, car l'homme du métier ne sait pas de prime abord si un tel composé tombe sous la revendication. Un nombre excessif d'essais serait nécessaire afin de tester aléatoirement chaque composé. La non-conformité avec les exigences quant au fond est telle qu'une recherche significative portant sur l'ensemble de l'objet revendiqué n'a pas pu être effectuée (règle 45 CBE et Directives B-VIII, 3.).
Par ailleurs, les séquences revendiquées ne sont aucunement distinguable des autres séquences de l'art antérieur, que celles-ci soient présentes naturellement dans la nature ou qu'elles aient été obtenues par une autre méthode que celle des revendications 1 ou 2.

Aucune recherche ne peut être effectué pour les revendications 3-10.

**ANNEXE AU RAPPORT DE RECHERCHE EUROPEENNE
RELATIF A LA DEMANDE DE BREVET EUROPEEN NO.**

EP 05 29 1780

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-06-2006

| Document brevet cité au rapport de recherche | | Date de publication | Membre(s) de la famille de brevet(s) | | Date de publication |
|---|---|---|---|---|---|
| WO 9827230 | A | 25-06-1998 | AU | 732146 B2 | 12-04-2001 |
| | | | AU | 5729298 A | 15-07-1998 |
| | | | CA | 2274319 A1 | 25-06-1998 |
| | | | EP | 0946755 A1 | 06-10-1999 |
| | | | JP | 2001506855 T | 29-05-2001 |
| | | | US | 6303344 B1 | 16-10-2001 |
| | | | US | 6653072 B1 | 25-11-2003 |
| | | | US | 6455253 B1 | 24-09-2002 |
| | | | US | 6579678 B1 | 17-06-2003 |
| | | | US | 6586182 B1 | 01-07-2003 |
| WO 9815567 | A | 16-04-1998 | AU | 4635597 A | 05-05-1998 |
| | | | DE | 69711667 D1 | 08-05-2002 |
| | | | EP | 0948626 A2 | 13-10-1999 |
| | | | EP | 0935603 A2 | 18-08-1999 |
| | | | WO | 9815630 A2 | 16-04-1998 |
| | | | NZ | 335358 A | 23-02-2001 |
| WO 9832845 | A | 30-07-1998 | AT | 255163 T | 15-12-2003 |
| | | | AU | 745615 B2 | 28-03-2002 |
| | | | AU | 5771898 A | 18-08-1998 |
| | | | CA | 2278585 A1 | 30-07-1998 |
| | | | DE | 69820054 D1 | 08-01-2004 |
| | | | DE | 69820054 T2 | 09-09-2004 |
| | | | DK | 988378 T3 | 26-01-2004 |
| | | | EP | 0988378 A1 | 29-03-2000 |
| | | | ES | 2212260 T3 | 16-07-2004 |
| | | | JP | 3703499 B2 | 05-10-2005 |
| | | | JP | 2001508660 T | 03-07-2001 |
| | | | PT | 988378 T | 30-04-2004 |

EPO FORM P0460